# EUROPÄISCHE PATENTSCHRIFT

(11) **EP 0 764 633 B1**
(45) Veröffentlichungstag und Bekanntmachung des Hinweises auf die Patenterteilung: **03.01.2001**
(21) Anmeldenummer: 96114579.4
(22) Anmeldetag: 12.09.1996
(51) Int. Cl.: C07C 263/10, C07C 265/02, C07C 265/08

(54) **Verfahren zur Herstellung von Etherisocyanaten**
Process for the preparation of etherisocyanates
Procédé de préparation d'étherisocyanates

(30) Priorität: 25.09.1995 DE 19535506
(43) Veröffentlichungstag der Anmeldung: 26.03.1997
(73) Patentinhaber: BAYER AG, 51368 Leverkusen (DE)
(72) Erfinder: Richter, Frank, Dr., 51373 Leverkusen (DE); Pedain, Josef, Dr., 51061 Köln (DE); Nachtkamp, Klaus, Dr., 29664 Walsrode (DE); Flink, Andreas, 41539 Dormagen (DE)

(56) Entgegenhaltungen:
- EP-A- 0 289 840
- DE-B- 1 154 092
- FR-A- 1 578 622

## Beschreibung

Die Erfindung betrifft ein Verfahren zur Herstellung von Etherisocyanaten durch Phosgenierung von Etheraminen in der Gasphase.

Ethergruppen enthaltene Isocyanate sind seit langem bekannt (z.B. Annalen der Chemie, 562 (1949), 83 ff). Die Verbindungen sind jedoch in der Regel nur in schlechter Ausbeute und Reinheit zugänglich.

Bei der Umsetzung von Etheraminen werden häufig durch Etherspaltung chlorierte Produkte erhalten (US-A 3 267 122). So wird z.B. H₂N(CH₂)₃-O-(CH₂)₄-O-(CH₂)₃NH₂ bei der Umsetzung mit COCl₂ in OCN(CH₂)₃Cl gespalten (Zitat: Angew. Chem., A 59 (1949), 271). Nur aliphatische Etheramine deren HCl-Salze in Chlorkohlenwasserstoffen löslich sind, z.B. C₄H₉-O-(CH₂)₃NH₂, konnten unterhalb 80°C zu Etherisocyanaten umgesetzt werden (Annalen der Chemie, 562 (1949), 105). Die Ausbeute an Isocyanat beträgt nur 86 % d.Th. Außerdem war es notwendig, vor der Phosgenierung das Amin in das Aminhydrochlorid zu überführen. Methoxypropylamin-hydrochlorid spaltet bei 140-150°C in 1-Chlornaphthalin mit Phosgen zu ca. 80 % in 3-Chlorpropylisocyanat (Annalen der Chemie, 562 (1949), 104).

Aus der EP-A-028984 ist bekannt, cycloaliphatische Diamine durch Phosgenierung in der Gasphase in die entsprechenden Diisocyanate zu überführen.

Die FR-A 1578622 lehrt Polyethergruppen enthaltende Diisocyanate, die durch Umsetzung von Etheraminen im Lösungsmittel hergestellt werden.

Spezielle Etherisocyanate können durch einfache Basenphosgenierung in Ausbeuten bis zu ca. 80 % hergestellt werden (z.B. DE-A 1 154 092). Die Produkte weisen allerdings einen sehr hohen Restchlorgehalt (um 0,1 %) auf. Ein derartig hoher Chlorgehalt der Diisocyanate steht häufig einem problemlosen Einsatz der Produkte, beispielsweise für die Bereitstellung nicht vergilbender Lackrohstoffe, im Wege.

Weiterhin müssen hierbei die jeweiligen Hydrochloride der Amine bzw. Carbaminate eingesetzt werden. Die Handhabung derartiger, heterogener Reaktionsmischungen bereitet erhebliche Schwierigkeiten und steht einer problemlosen, kostengünstigen Produktion der Isocyanate entgegen.

Weiterhin sind Ethergruppen enthaltende (Poly)isocyanate durch nucleophile Substitution organischer Halogenide mit Metallcyanaten zugänglich (z.B. JP 50 036 424; Arch. d. Pharm., 302 (1969), 617; DE-A 2 031 291).

Der hierbei auftretende Salzanfall sowie die meist niedrigen Umsetzungsraten stehen einer industriellen Verwertung des Verfahrens, auch unter ökologischen Gesichtspunkten, im Wege.

Weiterhin wurde zur Darstellung von Ether(poly)isocyanaten die Umsetzung von Ether(poly)aminen mit niedermolekularen Alkylisocyanaten vorgeschlagen, wobei anschließend durch thermische Zersetzung der entstandenen Harnstoffe und Alkylaminabtrennung die Ether(poly)isocyanate erhalten werden ("Umisocyanatisierung", vgl. z.B. DE-A 32 32 917). Dieses Verfahren hat jedoch mehrere Nachteile: einerseits fällt ein Nebenprodukt an, das entsorgt werden muß, andererseits verbleiben, insbesondere wenn die Ether(poly)isocyanate nicht durch anschließende Reinigungsverfahren aufgearbeitet werden können, beträchtliche Harnstoffanteile im Produkt und schließlich ist die "Umisocyanatisierung" eine typische Gleichgewichtsreaktion und daher schwer quantitativ durchführbar.

Ethergruppen enthaltende Isocyanate können auch durch Curtius-Umlagerung entsprechender Carbonsäureazide hergestellt werden, (z.B. J. prakt. Chem., 335 (1993), 294 und dort zit. Literatur), jedoch nur im Labormaßstab.

Wie beispielsweise in der DE-A 1 165 580 dargelegt, sind Ethergruppen enthaltende Polyisocyanate u.a. unter dem Aspekt ihrer Anwendung auf dem Lack- und Beschichtungsmittelsektor durchaus interessant.

Der vorliegenden Erfindung lag deshalb die Aufgabe zugrunde, einen einfachen Zugang zu Ethergruppen enthaltenden Isocyanaten zu finden, der es ermöglicht, in hohen Ausbeuten, ohne nennenswerte Produktverluste, beispielsweise durch Etherspaltung in Kauf nehmen zu müssen, qualitativ hochwertige, Ethergruppen enthaltende Isocyanate zur Verfügung zu stellen.

Es wurde nun gefunden, daß Ethergruppen enthaltende Mono- und Polyamine in sehr guten Ausbeuten und mit hoher Reinheit zu den entsprechenden Isocyanaten umgesetzt werden können, ohne daß eine Etherspaltung eintritt, wenn diese in der Gasphase bei dem angewandten Druck im Temperaturbereich von 50-800°C, bevorzugt 100-550°C, in Abhängigkeit von ihrem Siedepunkt, gegebenenfalls in Gegenwart eines inerten Trägergases, mit Phosgen in der Gasphase umgesetzt werden.

Dies ist umso überraschender, da bekannt ist, daß die Etherspaltung der Etheramine bei höheren Temperaturen immer stärker in den Vordergrund tritt. So wird bei der Phosgenierung von 3-Methoxypropylamin in Toluol als Lösungsmittel unterhalb 110°C ein Gemisch von 3-Methoxypropylisocyanat und infolge Etherspaltung 3-Chlorpropylisocyanat gebildet, während bei höheren Temperaturen, beispielsweise bei 140-150°C in Chlornaphthalin als Lösungsmittel, im wesentlichen nur noch 3-Chlorpropylisocyanat entsteht (Annalen der Chemie, 562 (1949), 83).

In der DE-A 1 793 329 wird zwar eine Kaltphasen-Heißphasen-Phosgenierung in Lösung zur Herstellung von Ether(poly)isocyanaten vorgeschlagen, wobei angeblich keine oder nur wenig Etherspaltung auftritt, die realisierten Ausbeuten an Isocyanat belaufen sich jedoch nur auf 60-75 % d.Th., sofern es sich um ein (durch Destillation) erhaltenes Produkt handelt. Der Chlorgehalt der Produkte liegt mit 400-2000 ppm außerdem für viele Anwendungen, insbesondere auf dem Lack- und Beschichtungsmittelsektor, viel zu hoch.

Gegenstand der Erfindung ist ein Verfahren zur Herstellung von Ether(poly)isocyanaten aus Ether(poly)aminen, dadurch gekennzeichnet, daß Ether(poly)amine mit der mindestens stöchiometrischen Menge, bezogen auf die NH₂-Gruppe(n), Phosgen bzw. entsprechenden Mengen eines Phosgen freisetzenden Stoffes umgesetzt werden, wobei die Reaktion nahe oder oberhalb des Siedepunktes des Ausgangs(poly)amin(gemisch)es bei dem angewandten Druck im Temperaturbereich von 50 bis 800°C, bevorzugt 100 bis 550°C, in der Gasphase durchgeführt wird.

Im erfindungsgemäßen Verfahren werden als Etheramine Verbindungen der Formel (I) eingesetzt

X(-R¹-O-R²-NH₂)ₙ (I),

wobei
- X: für H, NH₂ oder C(R³)₄₋ₙ steht,
- R¹, R² und R³: für gleiche oder verschiedene, gegebenenfalls verzweigte, gegebenenfalls mit Cl, Br, substituierte, gegebenenfalls Heteroatome (N, O, S) aufweisende C₁-C₁₀-Alkyl-, C₃-C₂₄-Cycloalkyl, C₇-C₂₄-Aralkyl-, C₆-C₂₄-Reste stehen und R¹ kann auch für eine direkte Bindung von X zum an R²-gebundenen Ethersauerstoffatom stehen
und
- n: für die Zahl 1, 2 oder 3 steht.

Das erfindungsgemäße Verfahren wird nach einer an sich bekannten Methode, z.B. nach der EP-A 0 570 799 oder der DE-A 4 412 327 durchgeführt. Die Reaktionspartner werden nahe oder oberhalb der Siedetemperatur des Ausgangsamin(gemisch)es in geeigneten Reaktoren eingeführt, vermischt und miteinander umgesetzt. Die Temperaturen hierfür liegen, abhängig vom gewählten Druck, zwischen 50 und 800°C, vorzugsweise zwischen 100 und 550°C. Das Verfahren wird in einem Druckbereich von 10 mbar bis 5 bar, vorzugsweise 200 mbar bis 3 bar durchgeführt.

Die Zuführung der Reaktionskomponenten bei der Gasphasenphosgenierung kann gegebenenfalls in Gegenwart bei der Phosgenierreaktion inerter Zusatzstoffe wie z.B. Trägergase, erfolgen. Als Trägergase können Stickstoff, Argon oder andere inerte Gase und Dämpfe technisch verfügbarer Lösungsmittel wie z.B. Chlorbenzol, Dichlorbenzole, Xylole, Chlornaphthaline, Decanhydronaphthalin etc. eingesetzt werden.

Das bei der Phosgenierung eingesetzte Phosgen wird, bezogen auf die primären Aminogruppen des Ausgangsamin(gemisch)es, stöchiometrisch oder im Überschuß eingesetzt. Im allgemeinen genügt eine Phosgenmenge die 100 - 300 % der Theorie, vorzugsweise 100 - 200 % der Theorie, beträgt.

Nach Umsetzung werden durch Abkühlung des Gasstromes bis zu einer Temperatur oberhalb der Zersetzungstemperatur der entsprechenden, intermediären Carbamidsäurechloride die erfindungsgemäßen Etherisocyanate erhalten und nach bekannten Verfahren wie z.B. Destillation, Kristallisation, Extraktion, Dünnschichtdestillation usw., rein isoliert, bzw. als Rohprodukt(lösung) erhalten.

Die Amine, die nach dem erfindungsgemäßen Verfahren in die entsprechenden Isocyanate überführt werden, können beispielsweise
1) durch Alkoxylierung von Wasser oder anderer, gegebenenfalls poly-, OH-funktioneller Verbindungen wie Alkohole, Phenole und/oder Carbonsäuren sowie anschließende Aminierung (z.B. FR-A 1 361 810),
2) durch Polymerisation von Tetrahydrofuran und, gegebenenfalls nach weiterer Umsetzung mit Alkylenoxid, anschließende Behandlung wie unter (1) beschrieben,
3) durch Cyanethylierung von Wasser und anschließende Hydrierung zum Bis (3-amino-propyl)ether (z.B. DRP 731 708) oder durch Cyanethylierung anderer, gegebenenfalls poly-, OH-funktioneller Verbindungen, insbesondere von Di- und Triolen, sowie anschließende Hydrierung
erhalten werden.

Die Verwendbarkeit von Ethergruppen enthaltenden Mono- sowie Polyaminen in der erfindungsgemäßen Phosgenierreaktion wird im wesentlichen durch deren Dampfdruck beim angewandten Druck festgelegt. Bei besonders hochsiedenden Verbindungen kann es deshalb von Vorteil sein, sie als Azeotrope mit anderen Stoffen in die Phosgenierreaktion einzubringen oder ein Trägergas für die Zufuhr der Aminkomponente in den Reaktionsraum einzusetzen.

Typische Beispiele geeigneter (Poly)amine der Formel (I), die rein oder als Gemische eingesetzt werden können sind:
- Alkyl-aminoalkylether wie Aminomethyl-methyl-ether, Aminomethylethylether, Aminomethyl-propylether (auch Isomere), 1-Aminoethylmethylether, 2-Aminoethyl-methylether, Aminopropyl-methylther (auch Isomere) usw.
- Diaminooxoalkane wie 1,1'-Bis(aminomethyl)ether, 1,1'-Bis(aminoethyl)ether, 1,2'-Bis(aminoethyl)ether, 2,2'-Bis(aminoethyl)ether sowie technische Gemische der drei letztgenannten Diamine, (Bis(amino-n-propyl)ether (alle Isomeren, gegebenenfalls als Gemisch), Bis(amino-iso-propyl)ether (alle Isomeren, gegebenenfalls als Gemisch), Amino-n-propyl-(amino-isopropyl)ether (auch Isomere, gegebenenfalls als Gemisch) usw.,
- Diamino-(poly)oxoalkane wie 1,8-Diamino-1,5,8-trimethyl-3,6-dioxaoctan, 1,11-Diamimo-1,5,8,11-tetramethylundecan sowie alle Isomeren der beiden letztgenannten Verbindungen mit vicinaler O-N-Verknüpfung in reiner Form oder als Gemisch (z.B. als technisches Jeffamin® D 230), 1,8-Diamino-3,6-dioxaoctan (z.B. als technisches Jeffamin® EDR 148), 1,10-Diamino-4,7-dioxadecan, 1,12-Diamino-4,9-dioxadodecan, 1,14-Diamino-3,10-dioxatetradecan, 1,13-Diamino-4,7,10-trioxatridecan und andere,
- Triamino-(poly)oxoalkane, z.B. 1,7-Diamino-2,6-dioxa-4-aminomethoxyheptan, 1-Amino-2-oxa-3,3-bis(aminomethoxy)hexan, 1,9-Diamino-3,7-dioxa-5-(1-amino-2-ethoxy)-nonan, 1-Amino-3-oxa-4,4-bis(1-amino-2-ethoxy)heptan, 1,11-Diamino-4,8-dioxa-6-(1-amino-5-oxabutyl)undecan, 1-Amino-4-oxa-5,5-bis(1-amino-5-oxabutyl)octan sowie Gemische der vorstehend genannten Mono-, Di- sowie Triamine.

Das nach dem erfindungsgemäßen Verfahren hergestellte Isomerengemisch aus 2-(2-Isocyanatopropoxy)-1-propylisocyanat, 1,1'-Oxydi-2-propylisocyanat und 2,2'-Oxydi-1-propylisocyanat ('Dipropylenglykoldiisocyant', Isomerengermisch) ist neu.

Die erfindungsgemäß hergestellten Etherisocyanate sind wertvolle Rohstoffe zur Herstellung von, gegebenenfalls geschäumten, Polyurethanen, Klebstoffen, Beschichtungsmitteln, Emulgatoren, Verdickungsmitteln, oligomeren Isocyanatmodifizierungsprodukten wie z.B. Uretdion-, Isocyanurat-, Carbodiimid-, Biuret-, Urethan- sowie Allophanat-haltigen Polyisocyanaten sowie von Hilfsmitteln wie sie z.B. für die Naßfestausrüstung von Papier und anderer Zelluloseprodukte verwendet werden. Sie finden u.a. Verwendung als Rohstoff zur Herstellung und/oder Formulierung von Wirkstoffen, Pharmazeutika u.a. wie z.B. in der DE-A 3 232 917 beschrieben.

### Beispiele

alle Prozentangaben beziehen sich auf das Gewicht.

### Beispiel 1

### 2-(2-Isocyanatopropoxy)-1-propylisocyanat, 1,1'-Oxydi-2-propylisocyanat und 2,2'-Oxydi-1-propylisocyanat ('Dipropylenglykoldiisocyanat', Isomerengemisch).

In ein auf 400°C erhitzes Mischrohr von 2,5 mm Durchmesser und 17,5 mm Länge mit nachgeschalteter Kondensationsstufe und anschließendem, Aktivkohlegefüllten COCl₂-Adsorptionsturm, strömen kontinuierlich durch eine in das Mischrohr ragende Düse 2,5 mol/h COCl₂, das in einem vorgeschalteten Wärmetauscher bei 950 mbar auf 420°C erwärmt wurde. Durch den Ringspalt zwischen Düse und Mischrohr werden simultan in einer Dosiergeschwindigkeit von 1 mol/h ein auf 320°C erwärmtes Amingemisch, erhalten durch katyltische Druckaminierung von technischem Dipropylenglykol (ca. 50 % 2-(2-Hydroxypropoxy)-1-propanol, ca. 40 % 1,1'-Oxydi-2-propanol und ca. 10 % 2,2'-Oxydi-1-propanol) mit einem Siedebereich von 72-78°C bei einem Druck von 7,5 mbar, sowie 0,1 mol/h trockener Stickstoff als Verdünnungsmittel in den Reaktionsraum eingeleitet. Durch Anlegen eines Unterdruckes am Ende der Kondensationsstufe wird im Mischrohr ein Druck von ca. 350 mbar aufrecht erhalten. Das heiße, den Reaktionsraum verlassende Reaktionsgemisch wird in einer Kondensationsstufe durch 1,2-Dichlorbenzol geleitet, das bei 150-160°C gehalten wird. Hierbei erfolgt die selektive Kondensation der gebildeten Diisocyanate. Aus dem die Waschstufe durchlaufenden, im wesentlichen Stickstoff, HCl und überschüssiges COCl₂ enthaltenden Gasgemisch wird in dem Adsorptionsturm COCl₂ abgetrennt. Das Diisocyanatgemisch wird destillativ in reiner Form gewonnen (Kp = 95°C/0,05 mbar, n_{D} = 1,4393/20°C) und fällt als farblose Flüssigkeit mit einem gemäß DIN 53 185 titrierten NCO-Gehalt von 45,4 % (theoret.: 45,6 %). Die Ausbeute an reinem, destillierten Diisocyanatgemisch beträgt 98,2 % der Theorie, bezogen auf eingesetztes Diamingemisch, bei einer gaschromatographisch bestimmten Reinheit von 99,7 % und einem Gehalt an hydrolysierbarem Chlor von 43 ppm.

### Beispiel 2

### 1,8-Diisocyanato-3,6-dioxaoctan

2,5 kg (16,87 mol) 1,8-Diamino-3,6-dioxaoctan der Fa. Aldrich bzw Jeffamin® EDR 148 werden wie in Beispiel 1 angegeben in das Diisocyanat überführt und letzteres wie angegebenen isoliert.
Ausbeute: 3360 g = 99,5 % d. Th., Reinheit (GC): 99,8 %, NCO-Gehalt gemäß DIN 53 185: 42,0 % (theoret.: 42,0 %), HC-Gehalt: 48 ppm. Kp: 95°C/0,5 mbar.

### Beispiel 3

### 1,12-Diisocyanato-4,9-dioxadodecan

2,5 kg (12,24 mol) 1,12 Diamino-4,9-dioxadocecan der Fa. Aldrich werden wie in Beispiel 1 angegeben in das Diisocyanat überführt und letzteres wie angegeben isoliert.
Ausbeute: 3056 g = 97,4 % d. Th., Reinheit (GC): 99,5 %, NCO-Gehalt gemäß DIN 53 185: 32,9 % (theoret.: 33,0 %), HC-Gehalt: 34 ppm. Kp: 83°C/0,2 mbar.

### Beispiel 4

### 1,3-Bis-(3-isocyanatopropoxy)-2,2-dimethylpropan

2,5 kg (11,45 mol) 1,3-Bis(3-aminopropoxy)-2,2-dimethylpropan werden wie in Beispiel 1 angegeben in das Diisocyanat überführt und letzteres wie angegeben isoliert.
Ausbeute: 3067 g = 99,1 % d. Th., Reinheit (GC): 99,8, NCO-Gehalt gemäß DIN 53 185: 31,0 % (theoret.: 31,1 %), HC-Gehalt: 24 ppm. Kp: 108°C/0,1 mbar.

### Beispiel 5

### 3-Methoxypropylisocyanat

1 000 g (11,2 mol) 3-Methoxypropylamin der Fa. Aldrich werden wie in Beispiel 1 angegeben in das Isocyanat überführt und letzteres wie angegeben isoliert.
Ausbeute: 1250 g = 96,8 % d. Th., Reinheit (GC): 99,1, NCO-Gehalt gemäß DIN 53 185: 36,5 % (theoret.: 36,50 %), HC-Gehalt: 44 ppm. Kp: 55°C /20 mbar.

Die Identität aller aufgeführten Verbindungen folgt weiterhin aus IR-, ¹H-NMR, ¹³C-NMR- sowie massenspektroskopischen Untersuchungen und den Resultaten der Elementaranalyse.

### Vergleichsbeispiele (Flüssigphosgenierung)

In einer Vierhalskolbenrührapparatur mit aufgesetztem Rückflußkühler, Innenthermometer, Tropftrichter und Einleitungsrohr werdem 440 g Monochlorbenzol bei 5°C mit 330 g Phosgen vermischt und anschließend eine Lösung von 71,5 g des in Beispiel 1 aufgeführten Diamingemisches in 900 g Monochlorbenzol über einen Zeitraum von 90 min zugetropft.

Anschließend wird unter Rühren langsam auf 90°C Innentemperatur bei gleichzeitiger Phosgeneinleitung (ca. 1 mol/h) erwärmt und mehrere Stunden bei dieser Temperatur gehalten. Ein völliges Aufklaren des Reaktionsgemisches konnte nicht erreicht werden. Nach Ausblasen des überschüssigen Phosgens mit Stickstoff, Filtration und destillativer Aufarbeitung wurden 19,5 g (19,6 % d.Th.) einer leicht gefärbten Flüssigkeit mit einem Siedebreich von 80-85°C/0,07 mbar und einem NCO-Gehalt gemäß DIN 53 185 von 45,2 % erhalten.

Weder durch Variation des Lösungsmittel (1,2-Dichlorbenzol bzw. Toluol: 34 % bzw. 22,3 d.Th. an Diisocyanat erhalten) noch durch Überführung des Diamingemisches in das Dihydrochlorid bzw. Bis(carbaminat) (15,3 bzw 27,6 % an Diisocyanat erhalten) konnte die Diisocyanatausbeute wesentlich erhöht werden. Der Restchlorgehalt der Produkte lag in keinem Fall unter 0,1 %.

Weitere Beispiele für Phosgenierungen von Etherdiaminen in flüssiger Phase sind z.B. in Annalen der Chemie, 562 (1949), 6 ff; DE-A 1 154 092; JP 4 027 365; FR 1 578 622 beschrieben.

## Patentansprüche

1. Verfahren zur Herstellung von Ether(poly)isocyanaten aus Ether(poly)aminen, dadurch gekennzeichnet, daß Ether(poly)amine mit der mindestens stöchiometrischen Menge, bezogen auf die NH₂-Gruppe(n), Phosgen bzw. entsprechenden Mengen eines Phosgen freisetzenden Stoffes umgesetzt werden, wobei die Reaktion nahe oder oberhalb des Siedepunktes des Ausgangs(poly)amin(gemisch)es bei dem angewandten Druck im Temperaturbereich von 50 bis 800°C in der Gasphase durchgeführt wird.

2. Verfahren nach Anspruch 1, dadurch gekennzeichnet, daß als Etheramine Verbindungen der Formel (I)
X(-R¹-O-R²-NH₂)ₙ (I)
eingesetzt werden, wobei
X für H, NH₂ oder C(R³)₄₋ₙ steht,
R¹, R² und R³ gleiche oder verschiedene, gegebenenfalls verzweigte, gegebenenfalls substituierte, gegebenenfalls Heteroatome (N, O, S) aufweisende C₁-C₁₀-Alkyl-, C₃-C₂₄-Cycloalkyl, C₇-C₂₄-Aralkyl-, C₆-C₂₄-Reste stehen und R¹ kann auch für eine direkte Bindung von X zum an R²-gebundenen Ethersauerstoffatom stehen
und
n für die Zahl 1, 2 oder 3 steht.

3. Isomerengemisch erhältlich nach Anspruch 1, dadurch gekennzeichnet, daß es aus 2-(2-Isocyanatopropoxy)-1-propylisocyanat, 1,1'-Oxydi-2-propylisocyanat und 2,2'-Oxydi-1-propylisocyanat ('Dipropylenglykoldiisocyanat', Isomerengemisch) besteht.

4. Verwendung des Isomerengemisches gemäß Anspruch 3 zur Herstellung von gegebenenfalls geschäumten Polyurethan-Kunststoffen, Lacken und Beschichtungsmitteln sowie als Zwischenprodukt zur Herstellung oder Formulierung von Wirkstoffen.

## Claims

1. Method for the preparation of ether (poly)isocyanates from ether (poly)amines, characterised in that ether (poly)amines are reacted with at least the stoichiometric quantity, referred to the NH₂ group(s), of phosgene or corresponding quantities of a substance releasing phosgene, with the reaction being carried out in the vapour phase close to or above the boiling point of the starting (poly)amine (mixture) at the applied pressure in the temperature range of from 50 to 800°C.

2. Method according to claim 1, characterised in that the ether amines used are compounds of formula (I)
X(-R¹-O-R²-NH₂)ₙ (I),
wherein
X represents H, NH₂ or C(R³)₄₋ₙ,
R¹, R² and R³ represent identical or different, optionally branched, optionally substituted, optionally heteroatom-containing (N, O, S) C₁-C₁₀ alkyl, C₃-C₂₄ cycloalkyl, C₇-C₂₄ aralkyl, C₆-C₂₄ radicals and R¹ can also represent a direct bonding of X to the ether oxygen atom bonded to R²
and
n represents the number 1, 2 or 3.

3. Mixture of isomers obtainable according to claim 1, characterised in that it consists of 2-(2-isocyanatopropoxy)-1-propyl isocyanate, 1,1'-oxydi-2-propyl isocyanate and 2,2'-oxydi-1-propyl isocyanate ("dipropylene glycol diisocyanate", mixture of isomers).

4. Use of the mixture of isomers according to claim 3 for the production of optionally foamed polyurethane plastics, of paints and coatings and as an intermediate product for the preparation or formulation of active substances.

## Revendications

1. Procédé pour la préparation d'éther-(poly)isocyanate à partir d'éther-(poly)amines, caractérisé en ce que l'on fait réagir les éther-(poly)amines avec la quantité au moins stoechiométrique, par rapport aux groupes NH₂, de phosgène ou la quantité correspondante d'une substance libérant du phosgène, la réaction étant réalisée au voisinage ou au-dessus du point d'ébullition de la polyamine ou du mélange de polyamines de départ à la pression observée dans l'intervalle de température de 50 à 800°C en phase gazeuse.

2. Procédé selon la revendication 1, caractérisé en ce que les éther-amines mises en oeuvre sont des composés de formule (I)
X(-R¹-O-R²-NH₂)ₙ (I)
dans laquelle
X représente H, NH₂ ou C(R³)₄₋ₙ.
R¹, R² et R³, ayant des significations identiques ou différentes, représentent des radicaux alkyle en C₁-C₁₀, cycloalkyle en C₃-C₂₄, aralkyle en C₇-C₂₄, des radicaux en C₆-C₂₄. éventuellement ramifiés, éventuellement substitués, contenant le cas échéant des hétéroatomes (N, O, S), R¹ pouvant également représenter une liaison directe reliant X à l'atome d'oxygène d'éther fixé sur R²
et
n est égal à 1, 2 ou 3.

3. Mélange d'isomères obtenus selon la revendication 1, caractérisé en ce qu'il consiste en le 2-(2-isocyanatopropoxy)-1-propylisocyanate, le 1,1'-oxydi-2-propylisocyanate et le 2,2'-oxydi-1-propylisocyanate ("dipropylèneglycoldiisocyanate", mélange d'isomères).

4. Utilisation du mélange d'isomères selon la revendication 3, pour la fabrication de résines synthétiques de polyuréthannes éventuellement gonflées en mousse, de peintures et vernis et de produits de revêtement, ainsi qu'en tant que produit intermédiaire de la préparation ou de la formulation de substances actives.
